(19)

Europäisches
Patentamt
European
Patent Office
Office européen
des brevets

(11) **EP 4 552 578 A1**

(12) **EUROPEAN PATENT APPLICATION**

(43) Date of publication:
**14.05.2025 Bulletin 2025/20**

(21) Application number: **24178667.2**

(22) Date of filing: **29.05.2024**

(51) International Patent Classification (IPC):
***A61B 6/03*** (2006.01) ***A61B 6/00*** (2024.01)
***A61B 6/50*** (2024.01)

(52) Cooperative Patent Classification (CPC):
**A61B 6/037; A61B 6/481; A61B 6/507;** A61B 6/501

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **09.11.2023 EP 23208708**

(71) Applicant: **MedTrace Pharma A/S
2970 Hørsholm (DK)**

(72) Inventors:
• **Harms, Hans
2970 Hørsholm (DK)**
• **Lubberink, Mark
2970 Hørsholm (DK)**

(74) Representative: **AWA Denmark A/S
Strandgade 56
1401 Copenhagen K (DK)**

(54) **ESTIMATION OF CEREBROVASCULAR RESERVE**

(57) The present invention relates to a method for non-invasive quantitative measurement of cerebrovascular reserve (CVR) and cerebral blood flow (CBF) with $^{15}O$ -water positron emission tomography (PET) in a human, and to a vasodilatant for use in said method.

**FIG.7**

EP 4 552 578 A1

**Description**

### FIELD OF THE INVENTION

The present invention relates to a method for non-invasive quantitative measurement of cerebrovascular reserve (CVR) and cerebral blood flow (CBF) with $^{15}O$-water positron emission tomography (PET).

### BACKGROUND OF THE INVENTION

Evaluation of cerebral perfusion is important for patients with cerebrovascular disease to diagnose the hemodynamic significance of vascular lesions and determine the optimal treatment tactics for the patient. Cerebrovascular reserve capacity (CVR) describes how far cerebral perfusion can increase from a baseline value after stimulation.

The reserve of cerebral blood flow (CBF) can be estimated by measuring cerebrovascular reactivity to external stimuli (Gupta 2012). There have been two main approaches to measuring CVR. One approach attempts direct cerebral blood flow measurements of the brain tissue with flow-sensitive imaging techniques such as positron emission tomography, nuclear medicine techniques, CT perfusion, or MR perfusion before and after a vasodilatory stimulus. The second approach involves transcranial Doppler measurement of flow velocities (typically in the middle cerebral artery) distal to a lesion both before and after a vasodilatory stimulus with the increase flow velocity considered a surrogate for CVR.

Vasodilatory stimuli include increasing levels of CO2 (such as with breath-holding or inhalation of CO2 gas mixtures) and pharmacological challenge in the form of various pharmacological agents possessing vasoactive properties. These drugs include acetazolamide (an inhibitor of carbonic anhydrase), which causes an acidosis and a significant increase in brain perfusion due to dilatation of intracranial arteries. Acetazolamide causes a decrease in the resistance of cerebral vessels, accompanied by an increase in CO2 level and corresponding decrease in pH in the blood. This substance increases cerebral blood flow markedly in unaffected vessels, whereas in areas where blood is supplied by stenotic or malformed vessels, the flow either increases slightly or remains unchanged (Mamontov 2020). It is used routinely in patients with atherosclerotic cerebrovascular disease.

In a systematic review and meta-analysis of 1061 independent CVR tests in 991 unique patients with carotid stenosis or occlusion with a mean follow-up of 32.7 months, baseline CVR impairment was associated with increased risk of stroke or transient ischemic attack (TIA). The findings suggest a positive relationship between baseline CVR impairment and future ischemic events with a pooled odds ratio (OR) suggesting that patients with impaired CVR are approximately 4 times more likely to develop stroke or TIA (Gupta 2012).

The gold standard for in-vivo CBF measurements is positron emission tomography (PET) with $^{15}O$-water, since $^{15}O$-water is freely diffusible, metabolically inert and has an uptake rate that is linear with blood flow up to high flow values. Measurement of CBF with $^{15}O$-water requires the acquisition of an arterial input function, which can only be obtained accurately using radioactivity measurements of continuously sampled blood from an indwelling arterial catheter, usually placed in the radial artery. This is an invasive, not completely risk-free and often painful procedure, which has hampered the clinical application of this method (Chim 2015, Mandel 1977, Wallach 2004, Everett 2009). As a result, measurement of CBF with $^{15}O$-water is not used often, and potentially critical diagnostic information is therefore not routinely obtained.

Hence, the availability of a safe, non-invasive, but still fully quantitative method to determine CBF and CVR using $^{15}O$-water and PET would enable widespread clinical use of quantitatively accurate CBF and CVR measurements.

### BRIEF DESCRIPTION OF DRAWINGS

Figure 1 - Correlation (top) and agreement (bottom) between true CVR and measured CVR using noise-free simulated data, using the 3-parameter implementation of equation 9 (left) and the corresponding 2-parameter implementation (right).

Figure 2 - Correlation (top) and agreement (bottom) between true CVR and measured CVR using noisy simulated data, using the 3-parameter implementation of equation 9 (left) and the corresponding 2-parameter implementation (right).

Figure 3 - Correlation (top) and agreement (bottom) between true CVR and measured CVR using noise-free simulated data including a 5% partial blood volume, using the 3-parameter implementation of equation 9 (left) and the corresponding 2-parameter implementation (right).

Figure 4 - Correlation (top) and agreement (bottom) between true CVR and measured CVR using noisy simulated data including a 5% partial blood volume, using the 3-parameter implementation of equation 9 (left) and the corresponding 2-parameter implementation (right).

Figure 5 - Correlation (top) and agreement (bottom) between true CVR and measured CVR using noisy simulated data including a 5% partial blood volume, using the 3-parameter implementation of equation 9 (left) and the corresponding 2-parameter implementation (right). Blood volume was now accounted for in the models by subtracting a noisy version of the original blood curve.

**Figure 6** - Correlation (top) and agreement (bottom) between true CVR and measured CVR using noisy simulated data including a 5% partial blood volume, using the 3-parameter implementation of equation 9 (left) and the corresponding 2-parameter implementation (right).

**Figure 7** - Typical fit of the proposed model to a total brain grey matter baseline time-activity curve. Three-(3p) and two-parameter (2p) fits overlap.

**Figure 8** - Left: CVR measured using the 2-parameter non-invasive model versus CVR measured using the invasive method. Right: Corresponding Bland-Altman plot showing non-significant bias.

## SUMMARY OF THE INVENTION

It has now surprisingly been found that the need for arterial cannulation and sampling to obtain quantitative CBF and CVR values with $^{15}$O-water can be obviated by a method which comprises two $^{15}$O-water PET scans based on a fixed amount of radioactivity being administered at a fixed injection rate.

It is known how to describe the tracer kinetic analysis of $^{15}$O-water using differential equations. Rearranging the differential equations describing the tracer kinetic analysis of $^{15}$O-water at baseline and during vasodilation, yields, when injection of the radioactivity is done in a controlled and reproducible manner, a solution, where the arterial input function is no longer required. Instead, the vasodilation data is described as a function of the baseline data and CVR, or vice-versa. Together with a controlled automated power injector, injecting a fixed amount of radioactivity at a fixed injection rate, the method allows for non-invasive measurement of CVR and CBF using $^{15}$O-water and PET. The method is valid using any intervention that changes blood flow, but only blood flow, between the two PET scans.

According to the method of the present invention, a 1st known amount of $^{15}$O-water is administered to the patient using a controlled bolus injection. A first PET scan of the brain is simultaneously initiated. After a period of about 15 minutes, a 2nd controlled bolus injection of a known amount of $^{15}$O -water is administered, and a second PET scan of the brain is simultaneously initiated. A vasodilatant such as azetazolamide 10 mg/kg) is administered approximately 15 minutes before the start of the second PET scan (but after the end of the first PET scan). After the second PET scan has been acquired, both PET acquisitions are reconstructed into a dynamic series using all appropriate corrections for quantitative PET. The activity concentrations in identical regions of interest in both scans are then measured and time-activity curves (TACs) are constructed showing the activity concentration over time. Finally, the combinations of TACs are fitted to a tracer kinetic model that describes one of the TACs as a function of the other.

Accordingly, in **a first aspect** of the invention there is provided a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1st PET acquisition of the brain, followed by

b) administering a bolus of a vasodilatant, 5-15 minutes before the second injection, to said human subject, followed by

c) administering **a second IV bolus $^{15}$O-water** to said human subject and simultaneously start a 2nd PET acquisition of the brain, followed by

d) reconstructing both PET acquisitions into dynamic series using all appropriate corrections for quantitative PET, followed by

e) measuring the activity concentrations in identical regions of interest in both scans and construct time-activity curves (TACs) showing the activity concentration over time, followed by

f) fitting the combinations of TACs to a tracer kinetic model that describes one of the TACs as a function of the other, $CBF/V_T$ (where $V_T$ is the water partition coefficient) and CVR,

g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

In **a second aspect** of the invention there is provided a vasodilatant for use in a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1st PET acquisition of the brain, followed by

b) administering a bolus of a vasodilatant, 5-15 minutes before the second injection, to said human subject, followed by

c) administering **a second IV bolus $^{15}$O-water** to said human subject and simultaneously start a 2$^{nd}$ PET acquisition of the brain, followed by

d) reconstructing both PET acquisitions into dynamic series using all appropriate corrections for quantitative PET, followed by

e) measuring the activity concentrations in identical regions of interest in both scans and construct time-activity curves (TACs) showing the activity concentration over time, followed by

f) fitting the combinations of TACs to a tracer kinetic model that describes one of the TACs as a function of the other, CBF/$V_T$ (where $V_T$ is the water partition coefficient) and CVR,

g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

In **a third aspect** of the invention there is provided a bolus composition comprising a first IV bolus $^{15}$O-water and a second IV bolus $^{15}$O-water, for use in a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1$^{st}$ PET acquisition of the brain, followed by

b) administering a bolus of a vasodilatant, 5-15 minutes before the second injection, to said human subject, followed by

c) administering **a second IV bolus $^{15}$O-water** to said human subject and simultaneously start a 2$^{nd}$ PET acquisition of the brain, followed by

d) reconstructing both PET acquisitions into dynamic series using all appropriate corrections for quantitative PET, followed by

e) measuring the activity concentrations in identical regions of interest in both scans and construct time-activity curves (TACs) showing the activity concentration over time, followed by

f) fitting the combinations of TACs to a tracer kinetic model that describes one of the TACs as a function of the other, CBF/$V_T$ (where $V_T$ is the water partition coefficient) and CVR,

g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

In **a fourth aspect** of the invention there is provided a bolus dosing system comprising a first IV bolus $^{15}$O-water and a second IV bolus $^{15}$O-water, for use in a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1$^{st}$ PET acquisition of the brain, followed by

b) administering a bolus of a vasodilatant, 5-15 minutes before the second injection, to said human subject, followed by

c) administering **a second IV bolus $^{15}$O-water** to said human subject and simultaneously start a 2$^{nd}$ PET acquisition of the brain, followed by

d) reconstructing both PET acquisitions into dynamic series using all appropriate corrections for quantitative PET, followed by

e) measuring the activity concentrations in identical regions of interest in both scans and construct time-activity curves (TACs) showing the activity concentration over time, followed by

f) fitting the combinations of TACs to a tracer kinetic model that describes one of the TACs as a function of the other, CBF/$V_T$ (where $V_T$ is the water partition coefficient) and CVR,

g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

**DEFINITIONS**

In the context of the present application the following definitions will be used:

CVR = Cerebrovascular reserve capacity
CBF = Cerebral blood flow
TIA = Transient ischemic attack
PET = Positron emission tomography
TACs = Time-activity curves
$V_T$ = water partition coefficient

15O-water or $^{15}$O-H$_2$O means oxygen-15 labelled water which is a radioactive variation of regular water in which the oxygen atom has been replaced by oxygen-15 ($^{15}$O), a positron-emitting isotope. Oxygen-15 decays with a half-life of about 2.04 minutes to nitrogen-15, emitting a positron. The positron quickly annihilates with an electron, producing two gamma rays of about 511 keV which are detectable using a PET scanner.

## DETAILED DESCRIPTION

State of the art when measuring CBF and CVR requires the use of an arterial input function using arterial cannulation. Many publications (e.g. Fung 2013, Okazawa 2018, Khalighi 2018, Kuttner 2021, Zanotti-Fregonara 2011) have evaluated the use of image-derived input functions, where the arterial input function is estimated directly from the PET images. This is hampered by the limited spatial resolution of PET which is comparable to the dimensions of the carotid arteries and prohibits accurate quantification. None of these methods have yet shown a satisfactory agreement and correlation with arterial sampling. In addition, the proposed methods are highly scanner- and image reconstruction algorithm dependent and cannot be generally applied. The use of arterial spin labelling (ASL) or dynamic susceptibility-weighted (DSC) contrast enhanced magnetic resonance imaging have been suggested for measurement of CVR, but thus far, agreement between ASL and $^{15}$O-water has not been high, and DSC requires injection with Gd-based contrast agents which is not tolerated well in all patients.

As described in the summary of invention, the inventors have now surprisingly developed a method for obtaining quantitative CBF and CVR values comprising two $^{15}$O -water PET scans, which method obviates the need for arterial cannulation and sampling.

The kinetics of $^{15}$O -water in brain tissue at baseline can be described by:

$$\frac{dC(t)}{dt} = F \times C_A(t) - \frac{F}{V_T} C(t) \qquad \text{(eq. 1)}$$

Here, C(t) is the radioactivity concentration in tissue (regions, voxels) over time, the so-called time-activity curve (TAC); F is CBF, $C_A(t)$ is the arterial input curve, and $V_T$ is the partition coefficient of water.

The solution of this equation is:

$$C(t) = F \times C_A(t) \otimes e^{-\frac{F}{V_T}t} \qquad \text{(eq. 2)}$$

By performing two measurements, at baseline and during vasodilation and fitting eq. 2, adding a fitted blood volume parameter, to each dataset separately, $F_1$ (baseline) and $F_2$ (during vasodilation) can be estimated.

Cerebrovascular reserve is then calculated as the ratio of vasodilated and baseline flow:

$$CVR = \frac{F_2}{F_1} \qquad \text{(eq. 3)}$$

Rearranging the differential equations describing the tracer kinetic analysis of $^{15}$O-water at baseline and during vasodilation yields, when injection of the radioactivity is done in a controlled and reproducible manner, a solution where the arterial input function is no longer required. Instead, the vasodilation data is described as a function of the baseline data and CVR, or vice-versa. Together with a controlled automated power injector, injecting a fixed amount of radioactivity at a fixed injection rate, the method allows for non-invasive measurement of CVR and CBF using $^{15}$O-water and PET:
Using

$$\dot{F}_2 = F_1 \times CVR \qquad (eq.\ 4)$$

eq. 2 can be rewritten as:

$$\frac{dC_2(t)}{dt} = F_1 \times CVR \times C_A(t) - \frac{F_1 \times CVR}{V_T} C_2(t) \qquad (eq.\ 5)$$

Multiplying eq. 1 with CVR gives:

$$CVR \times \frac{dC_1(t)}{dt} = CVR \times F_1 \times C_A(t) - CVR \times \frac{F_1}{V_T} \times C_1(t) \qquad (eq.\ 6)$$

Assuming an identical arterial input function $C_A(t)$ during both scans, subtraction of eq. 6 from eq. 5 yields:

$$\frac{dC_2(t)}{dt} - CVR \times \frac{dC_1(t)}{dt} = \frac{F_1 \times CVR}{V_T} \times C_1(t) - \frac{F_1 \times CVR}{V_T} \times C_2(t) \qquad (eq.\ 7)$$

which results in a differential equation for $C_2(t)$ *that no longer includes $C_A(t)$*:

$$\frac{dC_2(t)}{dt} = CVR \times \frac{dC_1(t)}{dt} + \frac{F_1 \times CVR}{V_T} \times C_1(t) - \frac{F_1 \times CVR}{V_T} \times C_2(t) \qquad (eq.\ 8)$$

This equation has the following analytical solution:

$$C_2(t) = CVR \times C_1(t) + \frac{F_1 \times CVR}{V_T} \times (1 - CVR) \times C_1(t) \otimes e^{-\frac{F_1 \times CVR}{V_T} t} \qquad (eq.\ 9)$$

Hence, the time-activity curve during vasodilation $C_2(t)$ can be described as a function of the baseline time-activity curve $C_1(t)$ and the three parameters $F_1$, CVR and $V_T$.

Substituting $C_2$ by $C_1$, $F_1$ and $C_1$ by $F_2$ and $C_2$, respectively, and CVR by 1/CVR, $C_1(t)$ can in a similar way be described as a function of $C_2(t)$, F and $V_T$. Since F and $V_T$ always appear as $F/V_T$ in the operational equation, fitting both of them is redundant and instead the system is reduced to a two-parameter solution.

Fitting eq. 9 to the measured tissue TAC during vasodilation using non-linear regression gives CVR and $F/V_T$. By assuming a fixed value for $V_T$, both $F_1$ and $F_2$ can be separately estimated.

Using this method at the single voxel level is time-consuming due to the required computing power, but eq. 8 can be integrated leading to a linear problem:

$$C_2(t) = CVR \times C_1(t) + \frac{F_1 \times CVR}{V_T} \int_0^t (C_1(\tau) - C_2(\tau))d\tau \qquad (eq.\ 10)$$

$$\frac{C_2(t)}{C_1(t)} = CVR + \frac{\frac{F_1 \times CVR}{V_T} \int_0^t (C_1(\tau) - C_2(\tau))d\tau}{C_1(t)} \qquad (eq.\ 11)$$

Evaluating the left-hand side and the integral part of the right-hand side for each image frame and plotting them against each other results in a curve through which a straight line with intercept CVR and slope $F_1 CVR/V_T$ can be fitted. This procedure can be implemented efficiently using generalized least squares and is suitable for performing the analysis on all pixels of a dynamic image set, resulting in parametric images showing CVR at the voxel level.

In addition, a basis function implementation of equation 9 can be implemented, which also allows for linearization of the solution and fast computation of parametric images.

Eq. 9 can be rewritten as:

$$C_2(t) = \theta_1 \times C_1(t) + \theta_2 \times C_1(t) \otimes e^{-\theta_3 t} \qquad \text{(eq. 12)}$$

$$\theta_2 = \frac{F_1 \times CVR}{V_T} \times (1 - CVR) \qquad \theta_3 = \frac{F_1 \times CVR}{V_T}$$

where $\theta_1 = CVR$, and .

A set of basis functions $BF_i$ can then be created using a pre-defined discrete set of $\theta_3$ values

$$BF_i(t) = C_1(t) \otimes e^{-\theta_{3,i} t} \qquad \text{(eq. 13)}$$

and eq. 12 can be rewritten as a linear equation for each basis function:

$$C_2(t) = \theta_1 \times C_1(t) + \theta_2 \times BF_i(t) \qquad \text{(eq. 14)}$$

Equation 14 can then be solved using linear least squares for each basis function $BF_i$, and the basis function for which the residual sum of squares is lowest defines which of $\theta_1$, $\theta_2$ and $\theta_3$ best describes the measured PET data. From these, CVR and F can then be determined. A typical preselected set of $\theta_3$ values would compare 100 values ranging from 0.1 to 2 min$^{-1}$.

Accordingly, in **a first aspect** of the invention there is provided a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1$^{st}$ PET acquisition of the brain, followed by
b) administering a bolus of a vasodilatant to said human subject, 5-15 minutes before the second injection followed by
c) administering **a second IV bolus $^{15}$O-water** to said human subject and simultaneously start a 2$^{nd}$ PET acquisition of the brain, followed by
d) reconstructing both PET acquisitions into dynamic series using all appropriate corrections for quantitative PET, followed by
e) measuring the activity concentrations in identical regions of interest in both scans and construct time-activity curves (TACs) showing the activity concentration over time, followed by
f) fitting the combinations of TACs to a tracer kinetic model that describes one of the TACs as a function of the other, CBF/$V_T$ (where $V_T$ is the water partition coefficient) and CVR,
g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).
The present disclosure thus relates to the estimation of the cerebrovascular blood flow (CBF) and cerebrovascular reserve (CVR) in a human subject by performing the method according to the first aspect.
The present disclosure also relates to a vasodilatant for use in a method for the estimation of the cerebrovascular blood flow (CBF) and cerebrovascular reserve (CVR) in a human subject.
In **a second aspect** of the invention there is therefore provided a vasodilatant for use in a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1$^{st}$ PET acquisition of the brain, followed by
b) administering a bolus of a vasodilatant, 5-15 minutes before the second injection, to said human subject, followed by
c) administering **a second IV bolus $^{15}$O-water** to said human subject and simultaneously start a 2$^{nd}$ PET acquisition of the brain, followed by
d) reconstructing both PET acquisitions into dynamic series using all appropriate corrections for quantitative PET, followed by
e) measuring the activity concentrations in identical regions of interest in both scans and construct time-activity curves (TACs) showing the activity concentration over time, followed by
f) fitting the combinations of TACs to a tracer kinetic model that describes one of the TACs as a function of the other, CBF/$V_T$ (where $V_T$ is the water partition coefficient) and CVR,
g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

The use according to the second aspect is not intended to have a therapeutic purpose.

The present disclosure also relates to a bolus composition comprising a first IV bolus $^{15}$O-water and a second IV bolus $^{15}$O-water for use in a method for the estimation of the cerebrovascular blood flow (CBF) and cerebrovascular reserve (CVR) in a human subject.

In **a third aspect** of the invention there is therefore provided a bolus composition comprising a first IV bolus $^{15}$O-water and a second IV bolus $^{15}$O-water, for use in a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1st PET acquisition of the brain, followed by
b) administering a bolus of a vasodilatant, 5-15 minutes before the second injection, to said human subject, followed by
c) administering **a second IV bolus $^{15}$O-water** to said human subject and simultaneously start a 2rd PET acquisition of the brain, followed by
d) reconstructing both PET acquisitions into dynamic series using all appropriate corrections for quantitative PET, followed by
e) measuring the activity concentrations in identical regions of interest in both scans and construct time-activity curves (TACs) showing the activity concentration over time, followed by
f) fitting the combinations of TACs to a tracer kinetic model that describes one of the TACs as a function of the other, CBF/$V_T$ (where $V_T$ is the water partition coefficient) and CVR,
g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

Finally, the present disclosure also relates to a bolus dosing system comprising a first IV bolus $^{15}$O-water and a second IV bolus $^{15}$O-water for use in a method for the estimation of the cerebrovascular blood flow (CBF) and cerebrovascular reserve (CVR) in a human subject.

In **a fourth aspect** of the invention there is therefore provided a bolus dosing system comprising a first IV bolus $^{15}$O-water and a second IV bolus $^{15}$O-water, for use in a method for non-invasive quantitative measurement of cerebrovascular reserve (CVR) and cerebral blood flow (CBF) in a human, which method comprises the following steps:

a) administering **a first IV bolus $^{15}$O-water** to a human subject and simultaneously start a 1st PET acquisition of the brain, followed by
b) administering a bolus of a vasodilatant, 5-15 minutes before the second injection, to said human subject, followed by
c) administering **a second IV bolus $^{15}$O-water** to said human subject and simultaneously start a 2nd PET acquisition of the brain, followed by
d) reconstructing both PET acquisitions into dynamic series using all appropriate corrections for quantitative PET, followed by
e) measuring the activity concentrations in identical regions of interest in both scans and construct time-activity curves (TACs) showing the activity concentration over time, followed by
f) fitting the combinations of TACs to a tracer kinetic model that describes one of the TACs as a function of the other, CBF/$V_T$ (where $V_T$ is the water partition coefficient) and CVR,
g) returning estimated (maximum-likelihood) parameter values of the pharmacokinetic model for the brain including cerebral blood flow (CBF) and an estimate of the cerebrovascular reserve (CVR),

wherein the start of the two PET acquisitions of steps a) and c) are interspaced by at least 6 half-lives of $^{15}$O (about 12 min).

In an embodiment of any of the aspects of the present invention the first and second IV bolus $^{15}$O-water are identical. In an embodiment each IV bolus $^{15}$O-water contains an activity of 400 $\pm$ 40 MBq. In a preferred embodiment each IV bolus $^{15}$O-water contains an activity of 400 $\pm$ 3 MBq.

In an embodiment of any of the aspects of the present invention the start of the two PET acquisitions of steps a) and c) are interspaced by at least 12 minutes, such as by 12 minutes, by 14 minutes, by 16 minutes or by at least 20 minutes .

In an embodiment of any of the aspects of the present invention the first and second IV bolus $^{15}$O-water each has a volume of 2 ml $\pm$ 0.1 ml. In a preferred embodiment the first and second IV bolus $^{15}$O-water each have a volume of 5 ml $\pm$ 0.5 ml.

In a preferred embodiment of any of the aspects of the present invention the first and second IV bolus $^{15}$O-water are each administered over 5 $\pm$ 1 seconds.

In a preferred embodiment of any of the aspects of the present invention each IV bolus $^{15}$O-water is administered as 5 mL $\pm$ 0.5 ml $^{15}$O-water at an injection speed of 1 mL/s followed by 35 mL $\pm$ 1 ml saline at an injection speed of 2 mL/s, using a power injector.

In another preferred embodiment of any of the aspects of the present invention the vasodilatant is azetazolamide, administered as an IV bolus injection of 10 mg/kg body weight.

In an embodiment of any of the aspects of the present invention, the PET scans following administration of the first and second bolus $^{15}$O-water are each performed as dynamic PET scans of the brain of a duration of 10 min $\pm$2 min.

In a preferred embodiment of any of the aspects of the present invention, the PET scans following administration of the first and second bolus $^{15}$O-water are each performed as dynamic PET scans of the brain, each having a duration of 4-10 min.

In another embodiment of any of the aspects of the present invention the dynamic series reconstruction comprises frames of 1x10, 8x5, 4x10, 2x15, 3x20, 2x30, 2x60 seconds duration.

In a preferred embodiment of any of the aspects of the present invention the extracted TACs are fitted to the following pharmacokinetic model (eq. 9):

$$C_2(t) = CVR \times C_1(t) + \frac{F_1 \times CVR}{V_T} \times (1 - CVR) \times C_1(t) \otimes e^{-\frac{F_1 \times CVR}{V_T}t}$$

wherein $C_2(t)$ is the time-activity curve (TAC) during vasodilation, $C_1(t)$ is the baseline TAC, $F_1$ is the CBF at baseline (i.e. $CBF_1$), $V_T$ is the water partition coefficient and CVR is the cardiovascular reserve.

Or, using $F_1 = CBF_1$,

$$C_2(t) = CVR \times C_1(t) + \frac{CBF_1 \times CVR}{V_T} \times (1 - CVR) \times C_1(t) \otimes e^{-\frac{CBF_1 \times CVR}{V_T}t}$$

In Eq.9, substituting $C_2$ by $C_1$, $F_1$ and $C_1$ by $F_2$ and $C_2$, respectively, and CVR by 1/CVR, leads to $C_1(t)$ in a similar way can be described as a function of $C_2(t)$, $F_2$ and $V_T$.

In another embodiment of any of the aspects of the present invention the extracted TACs are fitted to the following pharmacokinetic model:

$$C_1(t) = 1/CVR \times C_2(t) + \frac{CBF_2 \times CVR}{V_T} \times (1 - CVR) \times C_2(t) \otimes e^{-\frac{CBF_2 \times CVR}{V_T}t}$$

Alternatively to steps e) and f) of any of the aspects of the present invention, fit individual corresponding voxel TACs to the same tracer kinetic model or a linearization of this model to obtain maps of CBF/$V_T$ and CVR.

Steps a) - g) of any of the aspects of the present invention can conveniently be automated by employing a computing device comprising a computer readable storage medium containing instructions that, when executed by a processor, are configured to cause said computing device to execute or perform a method comprising said steps a) - g).

The general, or overall methods referred to above are based on a plurality of emission tomography images, such as positron emission tomography (PET) images wherein each image represents concentrations of a tracer that has been injected at a specific time. The PET system detects radiation emitted indirectly by the tracer, i.e. after a tracer has been injected in the blood flow, a time activity curve (TAC) can be obtained by observing the radiation in a pixel/voxel over time.

PET is the preferred imaging method according to the preferred embodiments of the invention.

The present disclosure further relates to a system for estimating the cerebrovascular reserve of a human based on two sets of a plurality of emission tomography images, such as positron emission tomography (PET) images of the brain, wherein the acquisition of the two sets of e.g. PET images is interspaced by the administration of a vasodilating agent to said human, such that the first set of e.g. PET images is acquired under normal ("baseline") conditions and the second set under conditions where the healthy intracranial arteries are dilated, said system being arranged to perform the method according to the method described in the present disclosure. By comparison of the two sets of e.g. PET images, regions of the brain which are supplied by stenotic or malformed blood vessels (which do not dilate significantly in response to the administered vasodilating agent) can be mapped/quantified. The cardiovascular reserve is next calculated as the ratio of vasodilated and baseline flow.

This means that preferably the system comprises an emission imaging device arranged to provide the emission tomography images; and a processor or other means arranged to perform the method for mapping and/or identifying regions of the brain which are supplied by stenotic or malformed blood vessels. Furthermore, a non-transitive, computer-readable storage device for storing instructions that, when executed by a processor, performs the various methods described hereinabove according to the present disclosure. The methods may be implemented as software that is readable and executable by for example a computer processor.

## EXPERIMENTAL

*Clinical data - comparison invasive vs. non-invasive*

12 subjects (2 healthy controls, 10 multiple-sclerosis patients) underwent $^{15}$O-water PET-CT scans at baseline and during acetazolamide challenge (10 mg/kg). Both scans consisted of a 10 min dynamic scan after administration of $400 \pm 40$ MBq $^{15}$O-water using a power injector (5 mL @ 1 mL/s followed by 35 mL saline @ 2 mL/s). Arterial blood was sampled continuously (3 mL/min) and measured using an on-line detector (Veenstra PBS 101, Comercer, Joure, The Netherlands). Images were corrected for frame-by-frame motion and volumes of interest were defined on a co-registered T1-MRI using a probabilistic template (PVElab) and transferred to all PET images to create time-activity curves. Each scan was then analysed separately using non-linear regression of the single-tissue compartment model with delay- and dispersion-corrected arterial input function and fitted blood volume parameter, as well as using the proposed non-invasive model.

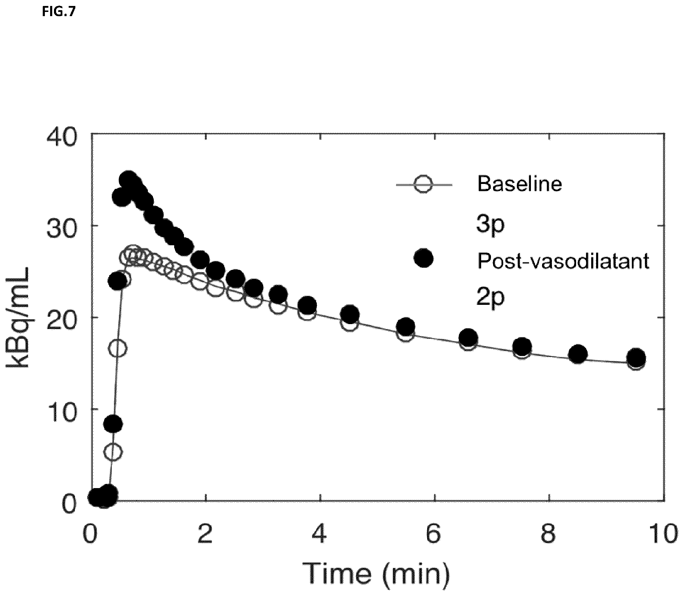

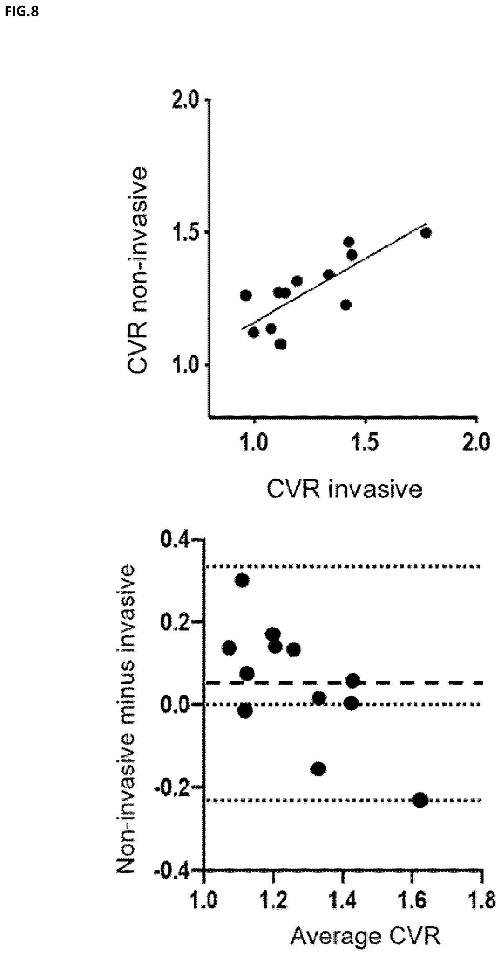

Figure 7 shows a typical fit of the proposed model to a total brain grey matter time-activity curve and figure 8 shows the relation between CVR based on separate arterial plasma-input model fits and CVR estimated using the proposed method.

*Simulations*

One hundred baseline time-activity curves (TACs) were simulated using equation (2) above, with CBF values chosen randomly in the interval between 0.3 and 0.7 mL/g/min and $V_T$ fixed to 0.85 mL/g. One hundred corresponding TACs after vasodilation were added using CVR chosen randomly between 1.0 and 1.6. Each pair of TACs was then analysed using equation 9, both with 3 and 2 parameters, and using equation 11 for the interval between 1 and 4 min after injection.

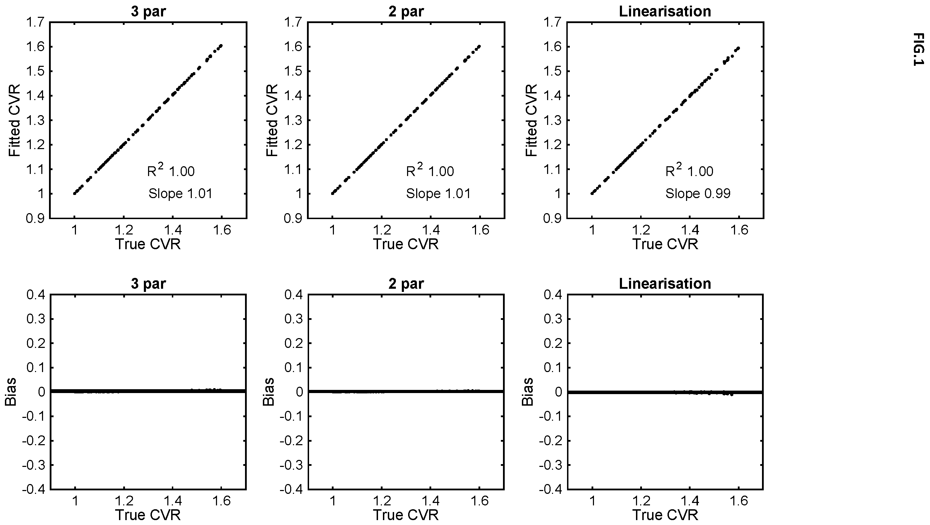

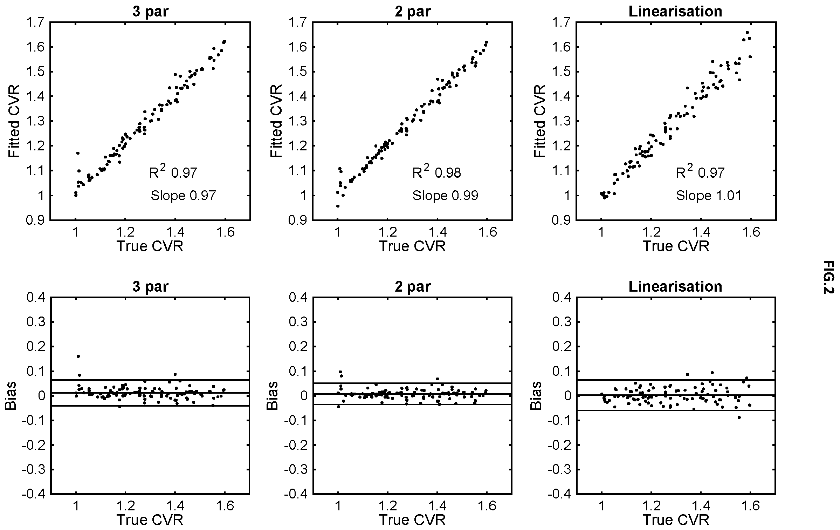

As shown in Figure 1, both 2- and 3-parameter models and the linearised version of the model produce nearly exactly the correct CVR when all assumptions are fulfilled, and data is noise-free. A random noise level typical of regional $^{15}$O-water PET data in the brain was added independently to each simulated TAC, and the simulation was repeated. Each pair of TACs was then again analysed using equation 9, both using 3 and 2 parameters, and using equation 11. As shown in Figure 2, both 2- and 3-parameter models result in an excellent correlation and agreement between true and estimated CVR.

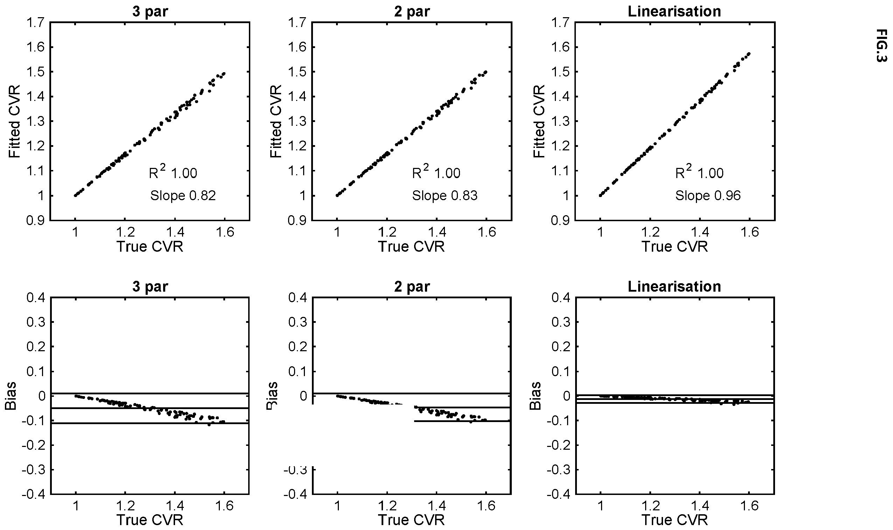

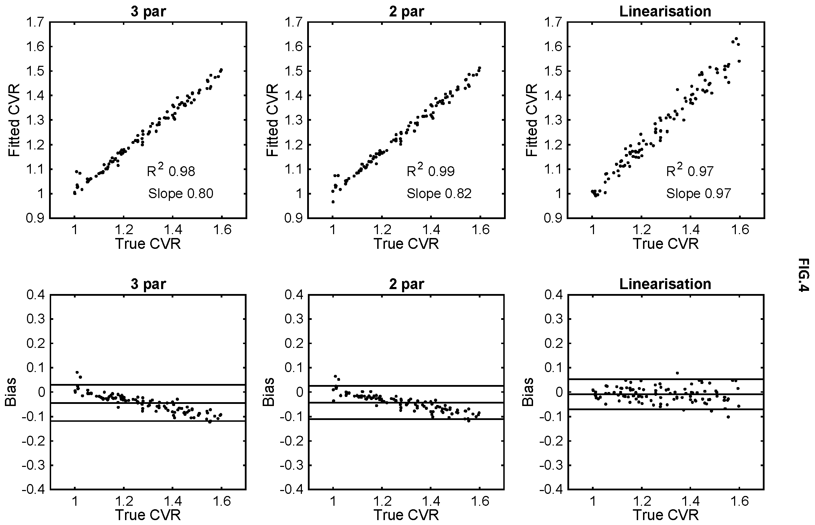

Effect of partial blood volume: Then, a typical blood volume of 5% was added and the simulations were repeated for noise-free (Figure 3) and noisy (Figure 4) data. As apparent in Figure 3, blood volume creates a negative bias proportional to CVR itself, but correlation is not affected. In addition, the linearised model appears to be affected less by blood volume than the full model.

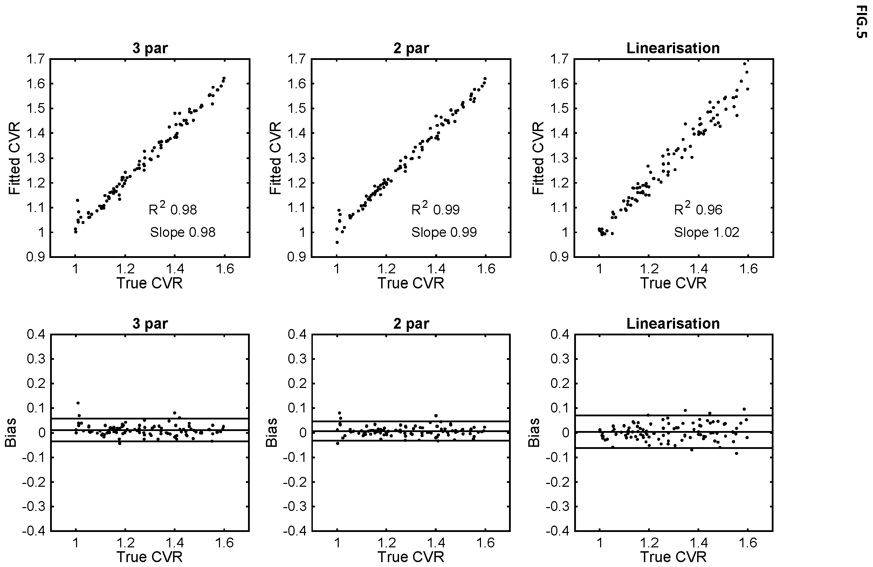

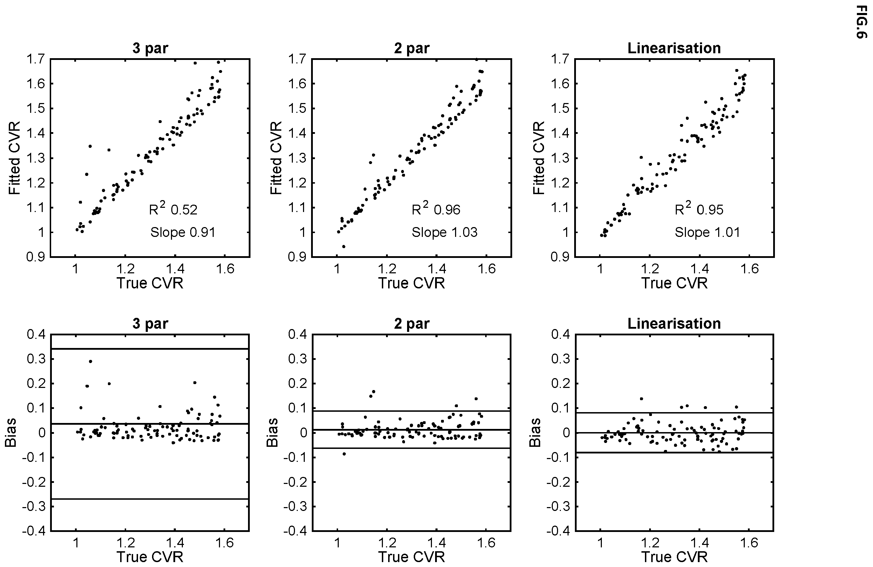

Correction for partial blood volume: Figures 5 and 6 represent two different ways of correcting for a blood volume component. First, the assumption was made that a noisy representation of the true input curve can be extracted from the PET data itself (e.g., using the carotid artery TAC). This noisy representation of the input curve was subtracted from the simulated tissue TACs prior to analysis, assuming a 5% blood volume, resulting in the correlations and biases shown in Figure 5. Second, another possible correction would be to subtract a population-average arterial input curve from the tissue TACs prior to analysis. To this end, the simulation was repeated with a different input curve for each simulated subject, with a $\pm 20\%$ variation in peak height, $\pm 20\%$ variation in peak clearance, and $\pm 10\%$ variation in tail clearance. This resulted in the correlations and biases shown in Figure 6.

## REFERENCES

1. Mamontov et al. Animal model of assessing functional reserve by imaging photoplethysmography. Nature Scientifc Reports | (2020) 10:19008 | https://doi.org/10.1038/s41598-020-75824-w

2. Gupta et al. Cerebrovascular Reserve and Stroke Risk in Patients with Carotid Stenosis or Occlusion. Stroke November 2012 p.2884 https://doi.org/10.1161/STROKEAHA.112.663716

3. Everett et al. Safety of Radial Arterial Catheterization in PET Research Subjects. J Nucl Med. 2009 October; 50(10): 1742. https://doi.org/10.2967/jnumed.109.063206

4. Acker et al. 15O-WATER PET IN NEGATIVE 99mTc-HMPAO SPECT. The Journal of Nuclear Medicine • Vol. 59 • No. 2 • February 2018

5. Chim et al. Complications related to radial artery occlusion, radial artery harvest, and arterial lines. Hand Clin. 2015;31(1):93-100.

6. Mandel et al. Radial artery cannulation and complications in 1,000 patients: precautions. J Hand Surg. 1977;2(6):482-5.

7. Wallach et al. Cannulation injury of the radial artery: diagnosis and treatment algorithm. Am J Crit Care Off Publ Am Assoc Crit-Care Nurses. 2004;13(4):315-9.

8. Everett et al. Safety of radial arterial catheterization in PET research subjects. J Nucl Med Off Publ Soc Nucl Med. 2009;50(10):1742.

9. Fung et al. Cerebral blood flow with [15O]water PET studies using an image-derived input function and MR-defined carotid centerlines. Phys Med Biol. 2013;58(6):1903-23.

10. Okazawa et al. Noninvasive method for measurement of cerebral blood flow using 0-15 water PET/MRI with ASL correlation. Eur J Radiol. 2018;105:102-9

11. Khalighi et al. Image-derived input function estimation on a TOF-enabled PET/MR for cerebral blood flow mapping. J Cereb Blood Flow Metab. 2018;38(1):126-35.

12. Kuttner et al. Cerebral blood flow measurements with 15O-water PET using a non-invasive machine-learning-derived arterial input function. J Cereb Blood Flow Metab. 2021;6:66.

13. Zanotti-Fregonara et al. Image-derived input function for brain PET studies: many challenges and few opportunities. J Cereb Blood Flow Metab Off J Int Soc Cereb Blood Flow Metab. 2011;31(10):1986-98.

**Claims**

1. A vasodilatant for use in a method for non-invasive quantitative measurement of cerebrovascular reserve (CVR) and cerebral blood flow (CBF) in a human, which method comprises:

   a) administering **a first IV bolus $^{15}$O-water** to a human subject and simultaneously start a 1$^{st}$ PET acquisition of the brain, followed by
   b) administering a vasodilatant to said human subject, 5-15 minutes before the second injection, followed by
   c) administering **a second IV bolus $^{15}$O-water** to said human subject and simultaneously start a 2$^{rd}$ PET acquisition of the brain, followed by
   d) reconstructing both PET acquisitions into a dynamic series using all appropriate corrections for quantitative PET, followed by
   e) measuring the activity concentrations in identical regions of interest in both scans and construct time-activity curves (TACs) showing the activity concentration over time, followed by
   f) fitting the combinations of TACs to a tracer kinetic model that describes one of the TACs as a function of the other, CBF/$V_T$ (where $V_T$ is the water partition coefficient) and CVR,
   g) returning estimated (maximum-likelihood) parameter values of the pharmacokinetic model for the brain including cerebral blood flow (CBF) and an estimate of the cerebrovascular reserve (CVR),

   wherein the start of the two PET acquisitions of steps a) and c) are interspaced by at least 6 half-lives of $^{15}$O (about 12 min).

2. A vasodilatant for use in a method according to claim 1 wherein the first and the second IV bolus $^{15}$O-water contain the same activity.

3. A vasodilatant for use in the method according to any one of claim 1 or 2 wherein each IV bolus $^{15}$O-water contains an activity of 400 ± 3 MBq.

4. A vasodilatant for use in the method according to any one of claim 1-3 wherein each IV bolus $^{15}$O-water has a volume of 5 ml ±0.5 ml.

5. A vasodilatant for use in the method according to any one of claim 1-4 wherein each bolus $^{15}$O-water is administered

over 5±1 seconds.

6. A vasodilatant for use in the method according to any one of claim 1-5 wherein each IV bolus $^{15}$O-water is administered as 5 mL ± 0.5 ml $^{15}$O-water at an injection speed of 1 mL/s followed by 35 mL ± 1 ml saline at an injection speed of 2 mL/s, using a power injector.

7. A vasodilatant for use in the method according to any one of claim 1-6 wherein the vasodilatant is azetazolamide, administered as an IV dose of 10 mg/kg body weight.

8. A vasodilatant for use in the method according to any one of claim 1-7 wherein the PET scans following administration of the first and second bolus $^{15}$O-water are each performed as dynamic PET scans of the brain of a duration of 10 min ±2 min.

9. A vasodilatant for use in the method according to any one of claim 1-8 wherein the dynamic series reconstruction comprises frames of 1x10, 8x5, 4x10, 2x15, 3x20, 2x30, 2x60 seconds duration.

10. A vasodilatant for use in the method according to any one of claim 1-9 wherein the corrections for quantitative PET are selected from decay, scatter, or dead-time.

11. A vasodilatant for use in the method according to any one of claim 1-10 wherein the time-activity curves (TACs) are fitted to a pharmacokinetic model:

$$C_2(t) = CVR \times C_1(t) + \frac{F_1 \times CVR}{V_T} \times (1 - CVR) \times C_1(t) \otimes e^{-\frac{F_1 \times CVR}{V_T}t}$$

wherein $C_2(t)$ is the TAC during vasodilation, $C_1(t)$ is the baseline TAC, $F_1$ is the cerebral flow at baseline (i.e. $CBF_1$), $V_T$ is the water partition coefficient and CVR is the cardiovascular reserve.

FIG.1

FIG.2

FIG.3

**FIG.4**

**FIG.5**

FIG.6

FIG.7

**FIG.8**

**Europäisches Patentamt**
**European Patent Office**
**Office européen des brevets**

# EUROPEAN SEARCH REPORT

Application Number

EP 24 17 8667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WILLIAMS MICHELLE C ET AL: "Computed tomography myocardial perfusion vs15O-water positron emission tomography and fractional flow reserve", EUROPEAN RADIOLOGY, SPRINGER BERLIN HEIDELBERG, BERLIN/HEIDELBERG, vol. 27, no. 3, 22 June 2016 (2016-06-22), pages 1114-1124, XP036151140, ISSN: 0938-7994, DOI: 10.1007/S00330-016-4404-5 [retrieved on 2016-06-22] * page 1116, right-hand column, last paragraph; figure 3a * | 1-11 | INV. A61B6/03 A61B6/00 A61B6/50 |
| X | LARSSON HENRIK BO ET AL: "Brain perfusion estimation by Tikhonov model-free deconvolution in a long axial field of view PET/CT scanner exploring five different PET tracers", EUROPEAN JOURNAL OF NUCLEAR MEDICINE AND MOLECULAR IMAGING, vol. 51, no. 3, 16 October 2023 (2023-10-16), pages 707-720, XP093205441, Berlin/Heidelberg ISSN: 1619-7070, DOI: 10.1007/s00259-023-06469-w * page 710, left-hand column, last paragraph * | 1-11 | |

TECHNICAL FIELDS SEARCHED (IPC)

A61B

The present search report has been drawn up for all claims

| Place of search | Date of completion of the search | Examiner |
|---|---|---|
| Munich | 16 September 2024 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MAMONTOV et al.** Animal model of assessing functional reserve by imaging photoplethysmography.. *Nature Scientifc Reports*, 2020, vol. 10, 19008, https://doi.org/10.1038/s41598-020-75824-w
- **GUPTA et al.** Cerebrovascular Reserve and Stroke Risk in Patients with Carotid Stenosis or Occlusion. *Stroke*, November 2012, 2884, https://doi.org/10.1161/STROKEAHA.112.663716
- **EVERETT et al.** Safety of Radial Arterial Catheterization in PET Research Subjects. *J Nucl Med.*, October 2009, vol. 50 (10), 1742, https://doi.org/10.2967/jnumed.109.063206
- **ACKER et al.** O-WATER PET IN NEGATIVE Tc-HMPAO SPECT. *The Journal of Nuclear Medicine •*, February 2018, vol. 59 (2)
- **CHIM et al.** Complications related to radial artery occlusion, radial artery harvest, and arterial lines. *Hand Clin.*, 2015, vol. 31 (1), 93-100
- **MANDEL et al.** Radial artery cannulation and complications in 1,000 patients: precautions. *J Hand Surg.*, 1977, vol. 2 (6), 482-5
- **WALLACH et al.** Cannulation injury of the radial artery: diagnosis and treatment algorithm. *Am J Crit Care Off Publ Am Assoc Crit-Care Nurses.*, 2004, vol. 13 (4), 315-9

- **EVERETT et al.** Safety of radial arterial catheterization in PET research subjects. *J Nucl Med Off Publ Soc Nucl Med.*, 2009, vol. 50 (10), 1742
- **FUNG et al.** Cerebral blood flow with [15O]water PET studies using an image-derived input function and MR-defined carotid centerlines. *Phys Med Biol.*, 2013, vol. 58 (6), 1903-23
- **OKAZAWA et al.** Noninvasive method for measurement of cerebral blood flow using 0-15 water PET/MRI with ASL correlation. *Eur J Radiol.*, 2018, vol. 105, 102-9
- **KHALIGHI et al.** Image-derived input function estimation on a TOF-enabled PET/MR for cerebral blood flow mapping. *J Cereb Blood Flow Metab.*, 2018, vol. 38 (1), 126-35
- **KUTTNER et al.** Cerebral blood flow measurements with O-water PET using a non-invasive machine-learning-derived arterial input function. *J Cereb Blood Flow Metab.*, 2021, vol. 6, 66
- **ZANOTTI-FREGONARA et al.** Image-derived input function for brain PET studies: many challenges and few opportunities. *J Cereb Blood Flow Metab Off J Int Soc Cereb Blood Flow Metab.*, 2011, vol. 31 (10), 1986-98